# EUROPEAN PATENT APPLICATION

(11) **EP 1 255 111 A1**
(43) Date of publication of application: **06.11.2002**
(21) Application number: 02007323.5
(22) Date of filing: 04.04.2002
(51) Int. Cl.: G01N 33/558, G01N 33/543

(54) **Immunochromato device and method for measuring samples using the same**

(30) Priority: 06.04.2001 JP 2001108156
(71) Applicant: MATSUSHITA ELECTRIC INDUSTRIAL CO., LTD., Kadoma-shi, Osaka 571-8501 (JP)
(72) Inventor: Kitawaki, Fumihisa, Kadoma-shi, Osaka 571-0064 (JP); Shigeto, Nobuyuki, Kyoto 610-0354 (JP)
(74) Representative: Schwabe - Sandmair - Marx

(57) **Abstract**

An immunochromatographic device includes a sample application section; a determination section; and a capillary-flow section. The determination section has an immobilization section including one type of first antibody specificallyreacting with all the types of target substances, or a plurality of types of first antibodies each specifically reacting with a corresponding type of target substance, the one type of first antibody or the plurality of types of first antibodies being immobilized. The sample application section or the capillary-flow section has a plurality of types of second antibodies each specifically reacting with all the types of target substances or a corresponding type of target substance. The plurality of types of second antibodies can be eluted. At least one type of second antibody specifically reacts with one type of target substance among the plurality of types of target substances. The plurality of types of second antibodies are labeled with different labeling substances.

## Description

### BACKGROUND OF THE INVENTION

### 1. FIELD OF THE INVENTION:

The present invention relates to an immunochromatography (immunochromato) device for use in a test method using dry chemistry, and a method for measuring samples using the same.

### 2. DESCRIPTION OF THE RELATED ART:

Recently, a variety of test methods have been utilized in clinical tests. One of them is a test method using dry chemistry. Dry chemistry is a method for measuring a target substance in a liquid sample by dropping the liquid sample on a reagent stored in a dry state in a developing layer matrix, such as, for example, a film or a test paper. This method can be carried out by a monolayer device or a multi-layer device. The monolayer device includes a single developing layer matrix for holding a reagent. The multi-layer device includes a combination of a developing layer, a reaction layer, a reagent layer, and the like as a developing layer matrix for holding a reagent. Both the monolayer and multi-layer devices, in which a reagent is already held on a developing layer matrix, have features in that (i) it is not necessary to adjust the reagent, (ii) the device is stored in a small space, and (iii) only a small amount of target substance is required. A representative test method using dry chemistry is immunochromatography. Immunochromatography is a test method which utilizes an antigen-antibody reaction and a capillary phenomenon. In a device for immunochromatography, a first antigen and a second antigen are held in a dry state on a carrier represented by a membrane filter. The first antigen is immobilized, and the second antigen is labeled with an indicator reagent. In a test, a test sample containing a target substance (antigen) is placed on the device and developed by a capillary phenomenon. Reaction sites are colored by sandwich-type antigen-antibody reactions, so as to identify an antigen, detect the presence or absence thereof, or measure the amount thereof. In addition to the sandwich type reaction, a competitive type reaction may be used as an alternative antigen-antibody reaction for immunochromatography. The structure of a device and a test method are the same as described above.

In addition to the above-described advantages of dry chemistry, a test method utilizing immunochromatography has the advantages of ease of handling, quick determination, and low cost. The test method is applicable to point of care testing (POCT) which has recently received attention, as well as clinical tests.

The antigen-antibody reaction is a specific equilibrium reaction occurring in vivo. In the antigen-antibody reaction, the ratio of (i) the logical product of the concentration of the antigen and the concentration of the antibody and (ii) the concentration of the antigen-antibody complex is kept constant at a given temperature. Accordingly, the concentration of the antigen-antibody complex to be generated is determined by the concentration of the antigen added to a given concentration of the antibody based on the equilibrium reaction. By preparing a calibration curve of the concentration of the antigen-antibody complex with respect to the concentration of the antigen, the unknown concentration of the antigen can be quantified.

According to multi-item immunochromatography, at least two immobilization sections, each including a first antibody immobilized therein, are provided in one immunochromatographic device, so that at least two types of antigens are simultaneously measured. The multi-item immunochromatography is performed as in a large-scale automation apparatus capable of performing multi-item measurement.

Simultaneous measurement of at least two types of antigens using immunochromatography has the following problem. When, for example, measuring two types of antigens simultaneously, samples are introduced to the device through a sample application section. Reaction first occurs in an immobilization section which is closer to the sample application section (upstream immobilization section). Then, reaction occurs in an immobilization section which is farther from the sample application section (downstream immobilization section). Before the reaction in the downstream immobilization section occurs, the antigen-antibody complex generated in the upstream immobilization section acts as a barrier which disturbs the flow flux of the sample sent downstream. The disturbance in the flow flux influences the reaction in the downstream immobilization section. As a result, the quantification capability of the immunochromatography of the concentration of the antigen is lowered.

### SUMMARY OF THE INVENTION

According to one aspect of the invention, an immunochromatographic device for detecting or measuring a plurality of types of target substances contained in a liquid sample is provided. The immunochromatographic device includes a sample application section; a determination section; and a capillary-flow section. The sample application section, the determination section and the capillary-flow section are located so that the liquid sample introduced to the sample application section is transferred to the determination section via the capillary-flow section. The determination section has an immobilization section including one type of first antibody specifically reacting with all the plurality of types of target substances, or a plurality of types of first antibodies each specifically reacting with a corresponding type of target substance, the one type of first antibody or the plurality of types of first antibodies being immobilized. The sample application section or the capillary-flow section has a plurality of types of second antibodies each specifically reacting with all the plurality of types of target substances or a corresponding type of target substance, wherein the plurality of types of second antibodies can be eluted. At least one type of second antibody specifically reacts with one type of target substance among the plurality of types of target substances. The plurality of types of second antibodies are labeled with different labeling substances.

In one embodiment of the invention, the plurality of target substances are glycohemoglobin and hemoglobin. The one type of first antibody is a first anti-hemoglobin monoclonal antibody specifically reacting with both glycohemoglobin and hemoglobin. The plurality of types of second antibodies include a second anti-hemoglobin monoclonal antibody specifically reacting with both glycohemoglobin and hemoglobin, and an anti-glycohemoglobin monoclonal antibody specifically reacting with glycohemoglobin.

In one embodiment of the invention, the labeling substances have different absorbance wavelengths from each other.

In one embodiment of the invention, the labeling substances have different fluorescence wavelengths from each other.

In one embodiment of the invention, the labeling substances have different phosphorescence wavelengths from each other.

According to another aspect of the invention, amethod for measuring a plurality of types of target substances using the above-described immunochromatographic device is provided. The method comprising the steps of (A) introducing a liquid sample to a sample application section; (B) measuring, in a determination section, one of hue, clarity, or brightness of complexes generated by reaction of the plurality of types of target substances, and a second antibody and a first antibody corresponding to each type of target substance; and (C) finding an absolute amount of each of the plurality of types of target substances or an amount ratio of the plurality of types of target substances contained in the liquid sample based on the measurements obtained in step (B).

Thus, the invention described herein makes possible the advantages of providing an immunochromatographic device for detecting or measuring at least two types of target substances with a high level of quantification capability.

These and other advantages of the present invention will become apparent to those skilled in the art upon reading and understanding the following detailed description with reference to the accompanying figures.

### BRIEF DESCRIPTION OF THE DRAWINGS

Figure **1** shows a structure of an immunochromatographic device according to an example of the present invention;
Figure **2** is a graph illustrating absorbance characteristics of an IMC-labeled second anti-hemoglobin antibody and an IC-labeled anti-glycohemoglobin antibody used in the immunochromatographic device shown in Figure **1**; and
Figure **3** is a graph illustrating the absorbance with respect to the ratio of glycohemoglobin in the glycohemoglobin-hemoglobin mixed solution.

### DESCRIPTION OF THE PREFERRED EMBODIMENTS

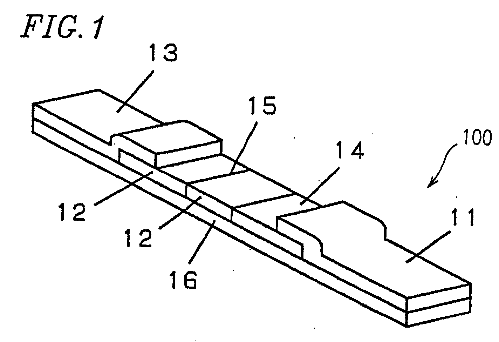

Figure **1** shows an immunochromatographic device **100** according to an example of the present invention. The immunochromatographic device **100** is used for detecting or measuring a plurality of types (for example, two types) of target substances contained in a liquid sample.

As shown in Figure **1**, the immunochromatographic device **100** includes a sample application section **11**, a determination section **12** and a capillary-flow section **14**, which are provided on a substrate **16**. The sample application section **11**, the determination section **12** and the capillary-flow section **14** are located so that a liquid sample introduced to the sample application section **11** is transferred to the determination section **12** via the capillary-flow section **14**. Reference numeral **13** refers to a water absorption section.

The determination section **12** has a line-like immobilization section **15**. The immobilization section **15** includes one type of first antibody specifically reacting with all the plurality of types of target substances, or a plurality of types of first antibodies each specifically reacting with a corresponding type of target substance. The one type of first antibody or the plurality of types of first antibodies are immobilized on an area of the determination section **12**.

On the sample application section **11** or the capillary-flow section **1**4, a plurality of types of second antibodies each specifically reacting with all the plurality of types of target substances or a corresponding type of target substance are provided in a state where the plurality of types of second antibodies can be eluted. At least one type of second antibody specifically reacts with one type of target substance among the plurality of types of target substances. The plurality of types of second antibodies are labeled with different labeling substances.

Due to such a structure, a plurality of antibody-antigen-antibody complexes are generated in the immobilization section **15**. Each antibody-antigen-antibody complex includes one type of target substance, and the second antibody and the first antibody corresponding to the target substance. Namely, the immunochromatographic device **100** includes a single immobilization section **15** where all the plurality of types of target substances are immobilized. Therefore, the flow flux of the liquid sample is more uniform than in the conventional device including a plurality of immobilization sections. Thus, the plurality of types of target substances can be detected or measured with a high level of quantification capability by detecting each labeling substance.

Exemplary liquid samples used in the present invention include bodily fluids, such as saliva, blood, hemolyzed blood, plasma, serum, urine, sweat, and tears.

Exemplary target substances include cell, protein, glycoprotein, enzyme, polysaccharide, bacterium, and virus.

A combination of a plurality of types of target substances contained in a sample of the present invention may be a combination of protein and protein having part of amino group termini glycosylated; for example, a combination of albumin and glycoalbumin, or a combination of hemoglobin and glycohemoglobin.

In the case where the combination of a plurality of types of target substances is a combination of glycohemoglobin and hemoglobin, the first antibody and the plurality of types of second antibodies may be as follows. The first antibody may be a first anti-hemoglobin monoclonal antibody specifically reacting with both glycohemoglobin and hemoglobin. The plurality of types of second antibodies may be a second anti-hemoglobin monoclonal antibody specifically reacting with both glycohemoglobin and hemoglobin, and an anti-glycohemoglobin monoclonal antibody specifically reacting with glycohemoglobin.

As a labeling substance, any substance which can label the second antibody is usable. Exemplary labeling substances include substances having optical characteristics; substances having magnetic particles such as iron oxide, aluminum oxide, and bacteria; substances having superconductive particles such as tin and fullerene; and radioactive substances. The substances having optical characteristics include substances having an absorbance wavelength in a specific wavelength range, substances having a fluorescence wavelength in a specific wavelength range, and substances having a phosphorescence wavelength in a specific wavelength range. Examples of substances having an absorbance wavelength in a specific wavelength range include aromatic compounds such as benzene, naphthalene, tetracene, rubrene, pyrene, and anthracene; aromatic compounds substituted with functional groups; particle markers such as gold colloid, silver colloid, selenium colloid, and colored latex; colorants having azo-based, quinone-based, triaryl-based, cyanine-based, phthalocyanine-based or indigo-based backbone structures; and particles containing the above-mentioned colorants. Examples of substances having a fluorescence wavelength in a specific wavelength range include aromatic compounds such as benzene, naphthalene, tetracene, rubrene and pyrene; aromatic compounds substituted with functional groups such as dansyl, and fluorescent compounds and fluorescent particles such as fluorescein, rhodamine, and coumarin. Examples of substances having a phosphorescence wavelength in a specific wavelength range include benzophenone.

A combination of labeling substances for labeling the plurality of types of second antibodies may be any combination of the substances having different absorbance wavelengths from each other. For example, a combination of pyrene and anthracene is usable. Using such a combination of labeling substances, the absorbance, reflection absorbance, or transmittance at the maximum absorbance wavelength of each of the labeling substances can be detected on a single immobilization section. Thus, a plurality of types of target substances can be detected or measured with a high level of quantification capability on a single immobilization section.

A combination of labeling substances for labeling the plurality of types of second antibodies may be any combination of the substances having different fluorescence wavelengths from each other. For example, a combination of pyrene and dansyl is usable. Using such a combination of labeling substances, the fluorescence strength, fluorescence life, or fluorescence quantum efficiency at the maximum fluorescence wavelength of each of the labeling substances with respect to a given exciting wavelength can be detected on a single immobilization section. Alternatively, the quenching effect caused by the interaction of the plurality of types of labeling substances can be detected. Thus, a plurality of types of target substances can be detected or measured with a high level of quantification capability on the single immobilization section.

A combination of labeling substances for labeling the plurality of types of second antibodies may be any combination of the substances having different phosphorescence wavelengths from each other.

According to an example of the present invention, a plurality of types of target substances are detected from a liquid sample as follows. The liquid sample is dropped onto the sample application section **11**, developed while the plurality of types of second antibodies are eluted, and caused to react with the first antibody (antibodies) in the immobilization section **15**. Based on a signal from labeling substances in the immobilization section **15**, the plurality of type of target substances contained in the liquid sample are detected.

More specifically, an example method for measuring a target substance according to the present invention includes the following steps A, B and C. In this example, a plurality of labeling substances having different absorbance wavelengths from each other in a visible wavelength range are used.

In step A, the liquid sample is introduced to the sample application section **11**.

In step B, in the determination section **12**, the hue, clarity, or brightness of the complexes generated by the reaction of the plurality of types of target substances, and the second antibodies and the first antibody (antibodies) corresponding to the target substances are measured.

In step C, the absolute amount of each of the plurality of types of target substances or the amount ratio of the plurality of types of target substances contained in the liquid sample is found based on the measurements obtained in step B.

A combination of labeling substances may be any combination of the substances having different absorbance wavelengths from each other in a visible wavelength range. Especially, a combination of a red colorant and a blue colorant is preferable.

According to a preferable method of the present invention, the hue, clarity, or brightness of the complexes are obtained as numerical values using a CCD camera or the like in step B. In step C, the numerical values are converted into physical amounts corresponding to the absolute amounts or the amount ratio by computer processing.

Herein, a visible wavelength range refers to a range of 350 nm to 750 nm.

Exemplary red colorants include a cyanine-based red colorant represented by general formula 1. Exemplary blue colorants include a cyanine-based blue colorant represented by general formula 2. 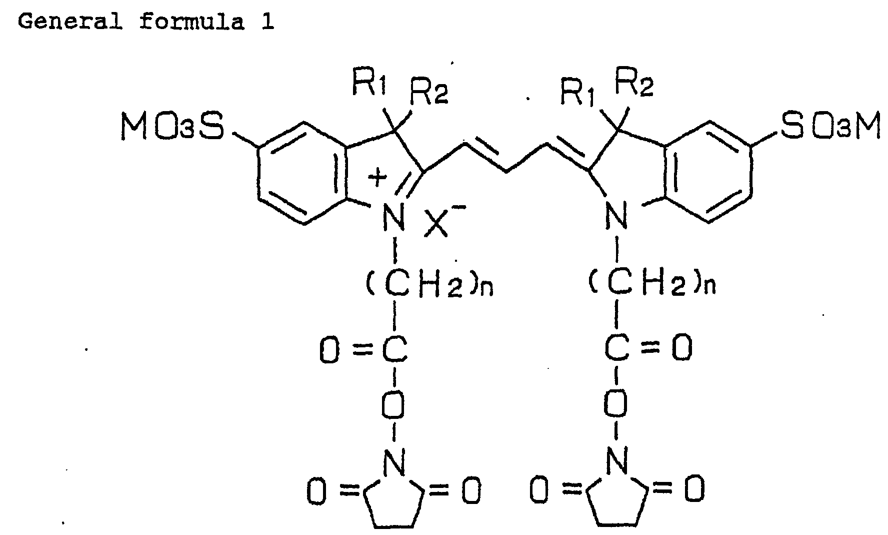 (where R₁ and R₂ are each hydrogen or an alkyl group; X is halogen; M is hydrogen or alkaline metal; and n is an integer of 1 through 4.) 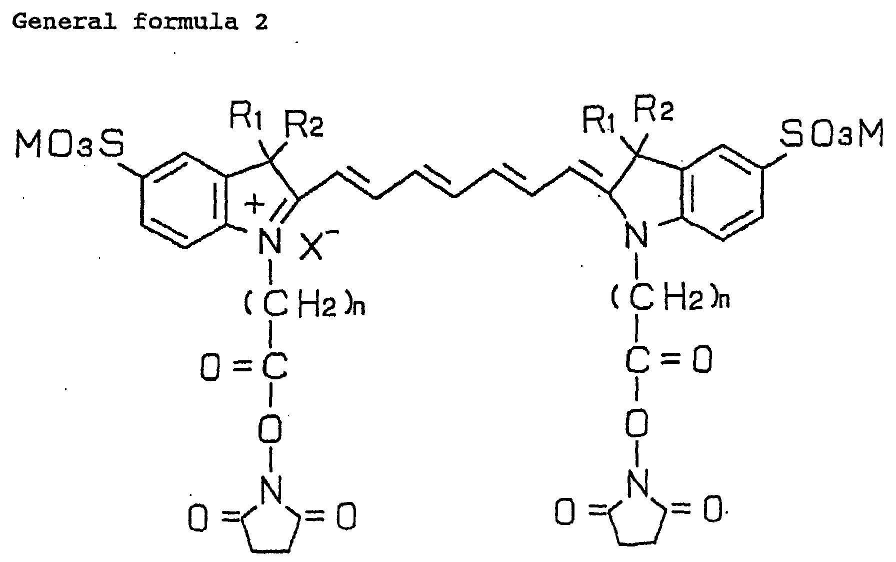 (where R₁ and R₂ are each hydrogen or an alkyl group; X is halogen; M is hydrogen or alkaline metal; and n is an integer of 1 through 4.)

General formula 3 shows an example of synthesis of the cyanine-based red colorant represented by general formula 1.

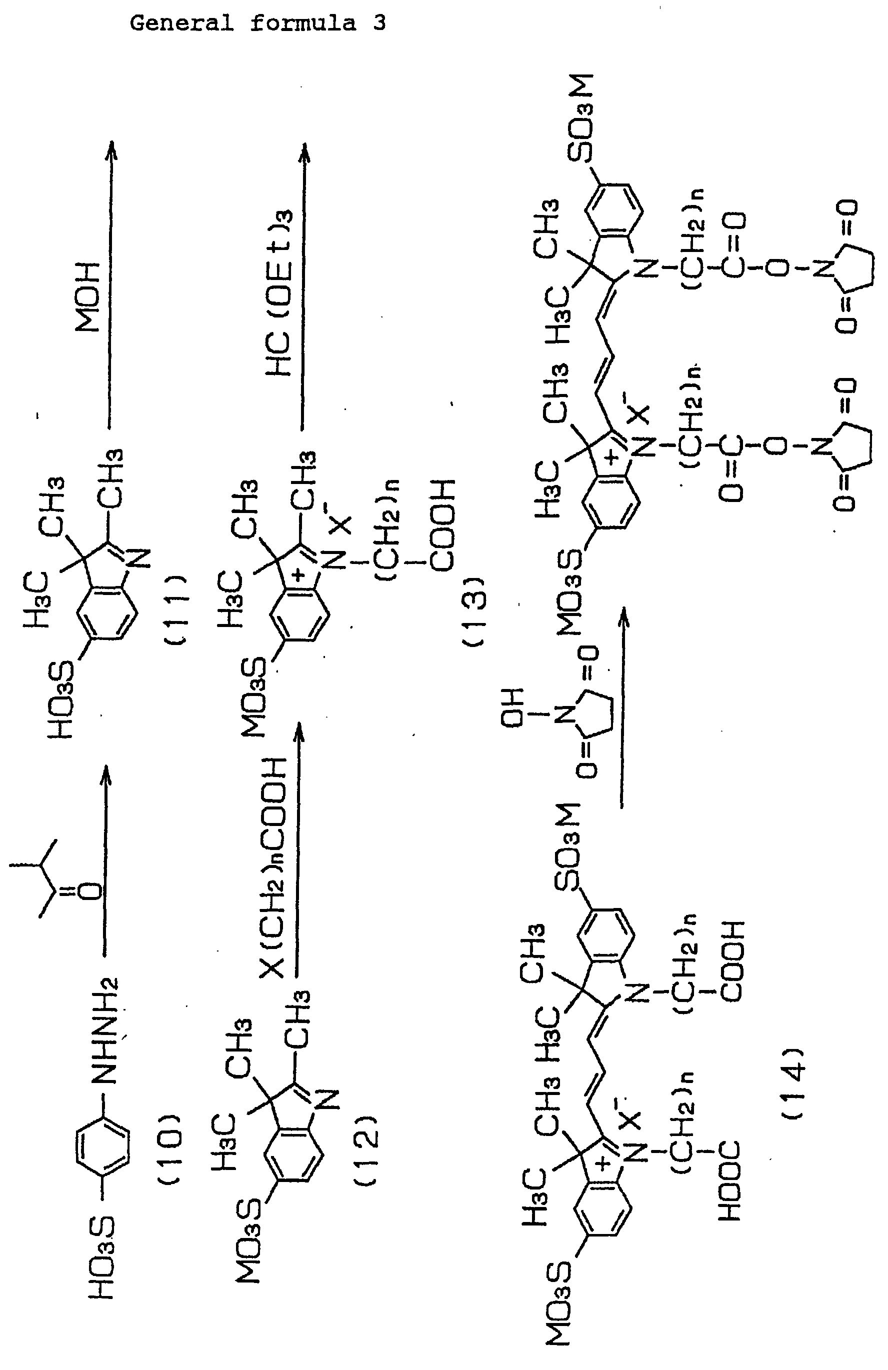

As shown in general formula 3, hydrazinobenzenesulfonic acid (10) and isopropylmethylketone are dissolved in an acidic solvent and heated, thereby preparing indolenium sulfonate (11). An alcoholic solution of indolenium sulfonate (11) is combined with a metal hydroxide-saturated alcoholic solution, thereby producing a metal salt of indolenium sulfonate (12).

Next, an organic solvent solution of the metal salt (12) is combined with halogenated alkyl acid, and the resultant substance is heated, thereby producing a metal salt of carboxyalkylindolenium sulfonate (13). The carbon number of the halogenated alkyl acid is preferably 1 through 4 in consideration of water-solubility thereof.

Then, the metal salt (13) and ethyl orthoformate are dissolved in a basic organic solvent and heated, thereby producing a carboxylic acid derivative (14). After this, an organic solvent solution of the carboxylic acid derivative (14) is combined with hydroxysuccinic acid imide and dicyclohexylcarbodiimide as a condensation agent, and the resultant substance is stirred, thereby producing the cyanine-based red colorant represented by general formula 1.

In order to synthesize the cyanine-based blue colorant represented by general formula 2, the above-described method is used except that glutaconaldehydetetramethylacetal is used instead of ethyl orthoformate.

Exemplary halogens contained in the compounds represented by general formula 1, general formula 2, formula 13 and formula 14 include fluorine, chlorine, bromine and iodine. Exemplary metals contained in the compounds represented by general formula 1, general formula 2, formula 12, formula 13 and formula 14 include lithium, sodium and potassium.

As the first and second antibodies, any antibody specifically reacting with the target substance or substances is usable. Exemplary antibodies include anti-cell antibodies, anti-protein antibodies, anti-glycoprotein antibodies, anti-enzyme antibodies, anti-polysaccharide antibodies, anti-bacterium antibodies, and anti-virus antibodies. Either monoclonal antibodies or polyclonal antibodies are usable.

As the first and second antibodies specifically reacting with one same type of target substance, any combination of antibodies which recognizes different antigen determinants of the target substance is usable.

The sample application section **11**, the capillary-flow section **14** and the determination section **12** may be formed of any material which can develop the liquid sample at an appropriate speed. For example, the sections **11, 12** and **14** may be formed of a porous carrier such as nitrocellulose or glass filter.

The immobilization section **15** is preferably line-shaped and provided on the determination section **12**.

### (Examples)

Hereinafter, a specific example of the present invention will be described. The present invention is not limited to the following specific example.

As the target substances, hemoglobin and glycohemoglobin were used. As the labeling substances, red colorant indolenine mero-cyanine (hereinafter, referred to as "IMC") and blue colorant indolenine cyanine (hereinafter, referred to as "IC") were used. IMC is represented by general formula 1, where X is iodine, M is potassium, and n (carbon number) is 2. IC is represented by general formula 2, where X is iodine, M is potassium, and n (carbon number) is 2.

As the first antibody, a first anti-hemoglobin antibody specifically reacting with both glycohemoglobin and hemoglobin was used. As the second antibodies, an anti-glycohemoglobin antibody specifically reacting with glycohemoglobin, and a second anti-hemoglobin antibody specifically reacting with both glycohemoglobin and hemoglobin, were used. The first anti-hemoglobin antibody and the second anti-hemoglobin antibody both specifically react with both glycohemoglobin and hemoglobin, but recognize different antigen determinants.

The immunochromatographic device **100** (Figure 1) according to the present invention was produced as follows.

First, an IMC-labeled second anti-hemoglobin antibody solution was prepared as follows. The second anti-hemoglobin antibody, which is one of the second antibodies, was dissolved in a phosphorus buffer solution (hereinafter, referred to as "PBS") so as to adjust the concentration thereof. The resultant solution (pH 7.4) was combined with an IMC solution (PBS, pH 7.4) and stirred for 24 hours. Then, a bovine serum albumin (hereinafter, referred to as "BSA") solution (pH 9.0) was added thereto. The resultant reaction mixture solution containing the IMC-labeled second anti-hemoglobin antibody was gel-filtered so as to remove unreacted antibodies and BSA therefrom. The purified IMC-labeled second anti-hemoglobin antibody was suspended in PBS, filtered by a 0.8 µm filter and stored at 4°C.

Next, an IC-labeled anti-glycohemoglobin antibody solution was prepared as follows. The anti-glycohemoglobin antibody solution (PBS, pH 7.4), which is one of the second antibodies, was combined with an IC solution (PBS, pH 7.4) and stirred for 24 hours. Then, a BSA solution (pH 9.0) was added thereto. The resultant reaction mixture solution containing the IC-labeled anti-glycohemoglobin antibody was gel-filtered so as to remove unreacted antibodies and BSA therefrom. The purified IC-labeled anti-glycohemoglobin antibody was suspended in PBS, filtered by a 0.8 µm filter, and stored at 4°C.

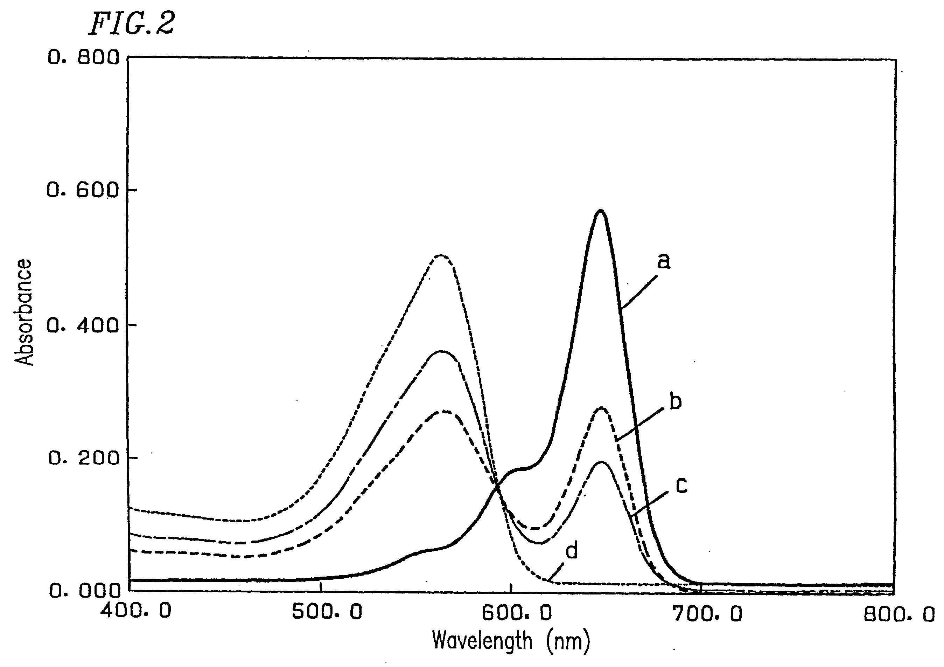

The absorbance characteristics of the IMC-labeled second anti-hemoglobin antibody and the IC-labeled anti-glycohemoglobin antibody were measured by a reflection absorbance spectrometer (CS9300, Shimadzu Corporation). The results are shown in Figure **2**. Curve a represents the result obtained from the IC-labeled anti-glycohemoglobin antibody. Curve b represents the result obtained from a 1:1 mixture solution of the IMC-labeled second anti-hemoglobin antibody and the IC-labeled anti-glycohemoglobin antibody. Curve c represents the result obtained from a 2:1 mixture solution of the IMC-labeled second anti-hemoglobin antibody and the IC-labeled anti-glycohemoglobin antibody. Curve d represents the result obtained from the IMC-labeled second anti-hemoglobin antibody. As can be appreciated from Figure **2**, the IMC-labeled second anti-hemoglobin antibody exhibits the maximum absorbance at 563 nm, and the IC-labeled anti-glycohemoglobin antibody exhibits the maximum absorbance at 646 nm.

The first anti-hemoglobin antibody was dissolved in PBS so as to adjust the concentration thereof. The resultant antibody solution was applied in the shape of a line to a central area of a nitrocellulose membrane by a solution injection apparatus and dried. Thus, the immobilization section **15** was formed. Then, the nitrocellulose membrane was immersed in a Tris-HCl buffer solution containing 1% skim milk and shaken for 30 minutes. Then, the nitrocellulose membrane was immersed in a Tris-HCl buffer solution (pH 8.2) and shaken for 10 minutes. The resultant nitrocellulose membrane was dried at room temperature.

The 1:1 mixture solution of the IMC-labeled second anti-hemoglobin antibody and the IC-labeled anti-glycohemoglobin antibody was applied to an area far from the immobilization section **15** of the resultant nitrocellulose membrane by the solution injection apparatus. Thus, the capillary-flow section **14** was formed.

The nitrocellulose membrane having the immobilization section **15** and the capillary-flow section **14**, the sample application section **11** formed of a nitrocellulose membrane, and the water absorption section **13** were bonded to the substrate **16** formed of white PET and having a thickness of 0.5 mm. The resultant assembly was cut into strips each having a width of 5 mm. Thus, the immunochromatographic device **100** was produced.

As the liquid samples, glycohemoglobin-hemoglobin mixture solutions having a ratio of glycohemoglobin of 4%, 5%, 6% and 7% were produced. These glycohemoglobin-hemoglobin mixture solutions were produced by mixing a hemoglobin solution and a glycohemoglobin solution having known concentrations.

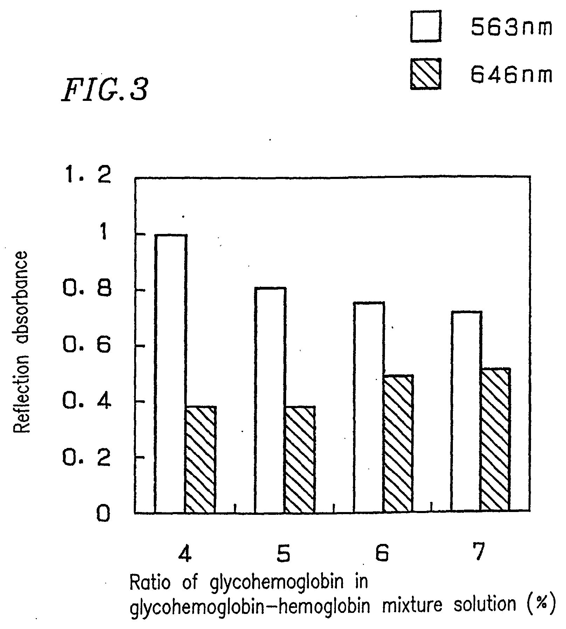

The reflection absorbance of the liquid samples was measured as follows. The glycohemoglobin-hemoglobin mixture solutions were each diluted four-folds, and 40 µl of each diluted solution was applied to the sample application section **11** of the immunochromatographic device **100**. The coloring of the immobilization section **15** five minutes after the application at 563 nm and 646 nm was measured by the reflection absorbance spectrometer (CS9300, Shimadzu Corporation). Figure 3 shows the results. From the results, an excellent relationship between the absorbance ratio at 563 nm and 646 nm and the amount ratio of glycohemoglobin and hemoglobin was found.

As can be appreciated, the concentration of glycohemoglobin and the concentration of hemoglobin in a glycohemoglobin-hemoglobin mixture solution can be measured with a high level of precision using the immunochromatographic device according to the present invention, by finding, beforehand, the concentration-reflection absorbance calibration curve of the samples including only hemoglobin having a known concentration and samples including only glycohemoglobin having a known concentration.

As described above, the present invention provides an immunochromatographic device for detecting or measuring at least two types of target substances with a high level of quantification capability.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

## Claims

1. An immunochromatographic device for detecting or measuring a plurality of types of target substances contained in a liquid sample, the immunochromatographic device comprising:
a sample application section;
a determination section; and
a capillary-flow section,
wherein:
the sample application section, the determination section and the capillary-flow section are located so that the liquid sample introduced to the sample application section is transferred to the determination section via the capillary-flow section,
the determination section has an immobilization section including one type of first antibody specifically reacting with all the plurality of types of target substances, or a plurality of types of first antibodies each specifically reacting with a corresponding type of target substance, the one type of first antibody or the plurality of types of first antibodies being immobilized,
the sample application section or the capillary-flow section has a plurality of types of second antibodies each specifically reacting with all the plurality of types of target substances or a corresponding type of target substance,
wherein the plurality of types of second antibodies can be eluted,
at least one type of second antibody specifically reacts with one type of target substance among the plurality of types of target substances, and
the plurality of types of second antibodies are labeled with different labeling substances.

2. An immunochromatographic device according to claim 1,
wherein:
the plurality of target substances are glycohemoglobin and hemoglobin,
the one type of first antibody is a first anti-hemoglobin monoclonal antibody specifically reacting with both glycohemoglobin and hemoglobin, and
the plurality of types of second antibodies include a second anti-hemoglobin monoclonal antibody specifically reacting with both glycohemoglobin and hemoglobin, and an anti-glycohemoglobin monoclonal antibody specifically reacting with glycohemoglobin.

3. An immunochromatographic device according to claim 1,
wherein the labeling substances have different absorbance wavelengths from each other.

4. An immunochromatographic device according to claim 1,
wherein the labeling substances have different fluorescence wavelengths from each other.

5. An immunochromatographic device according to claim 1,
wherein the labeling substances have different phosphorescence wavelengths from each other.

6. A method for measuring a plurality of types of target substances using an immunochromatographic device according to claim 3, the method comprising the steps of:
(A) introducing a liquid sample to a sample application section;
(B) measuring, in a determination section, one of hue, clarity, or brightness of complexes generated by reaction of the plurality of types of target substances, and a second antibody and a first antibody corresponding to each type of target substance; and
(C) finding an absolute amount of each of the plurality of types of target substances or an amount ratio of the plurality of types of target substances contained in the liquid sample based on the measurements obtained in step (B).
